Europäisches Patentamt

European Patent Office

Office européen des brevets

(11) Veröffentlichungsnummer: **0 287 896**
**A1**

## EUROPÄISCHE PATENTANMELDUNG

(21) Anmeldenummer: 88105507.3

(22) Anmeldetag: 07.04.88

(51) Int. Cl.⁴: **A61K 35/39** , **A01N 1/00** , **A61M 1/00**

(30) Priorität: 21.04.87 CH 1520/87

(43) Veröffentlichungstag der Anmeldung:
26.10.88 Patentblatt 88/43

(84) Benannte Vertragsstaaten:
AT BE CH DE ES FR GB GR IT LI LU NL SE

(71) Anmelder: Huber, Walter, Dr.
Sonnenbergstrasse 33
CH-8424 Embrach(CH)

(72) Erfinder: Huber, Walter, Dr.
Sonnenbergstrasse 33
CH-8424 Embrach(CH)

(74) Vertreter: EGLI-EUROPEAN PATENT
ATTORNEYS
Horneggstrasse 4
CH-8008 Zürich(CH)

(54) Verfahren und Einrichtung zur Gewinnung endokriner Inseln aus einem Spenderorgan sowie Verwendung der Inseln zur Behandlung von Stoffwechselkrankheiten.

(57) Die Erfindung bezieht sich auf ein Verfahren und eine Einrichtung zur Gewinnung endokriner Inseln aus einem Spenderorgan, zum Beispiel aus einer Bauchspeicheldrüse (Pankreas) sowie auf die Verwendung der gewonnenen Inseln.

Das Spenderorgan wird hierbei während einer Perfusionsphase mit einer gemischten Blut-Substanz künstlich durchblutet und anschliessend während einer Dissolutions-oder Digestionsphase bis zur Abscheidung des Fasermaterials in Collagenase-Flüssigkeit eingetaucht bis sich eine homogene Fragment-Suspension bildet.

Die Fragment-Suspension wird zur Gewinnung eines Absatzes (Sediment) aus exokrinem Zellmaterial und endokrinen Zellen zentrifugiert, so dass bei einer Insel-Isolationsphase die toten, exokrinen Zellanteile von den lebenden, endokrinen Inseln getrennt werden können. Die atraumatisch gewonnenen Inseln können zur Wiederaufnahme der normalen Stoffwechsel-Funktion eines Organs transplantiert oder injiziert werden.

FIG. 1

EP 0 287 896 A1

## Verfahren und Einrichtung zur Gewinnung endokriner Inseln aus einem Spenderorgan sowie Verwendung der Inseln zur Behandlung von Stoffwechselkrankheiten

Die Erfindung betrifft ein Verfahren und eine Einrichtung zur Gewinnung endokriner Zellen oder Inseln aus steril entnommenen Spenderorganen, insbesondere aus einer Bauchspeicheldrüse (Pankreas), wobei die Einrichtung ein Oxygenatorgerät, einen Gas-und Wärmeaustauscher, eine Heizvorrichtung sowie eine Sauerstoffzuführung umfasst.

Bei einem bekannten Verfahren zur Gewinnung von funktionsfähigen Inseln (Langerhans'sche Inseln) aus einem Pankreas wird unter Verwendung einer, aus Collagenase-, Trypsin-und Proteolyse-Anteilen bestehenden Lösungsmischung (Enzyme), oder aber unter duktaler Anwendung der vorstehenden, mit Foetal-Calf-Serum ergänzten Lösungsmischung, das Pankreas-Gewebe digestiv verarbeitet und dabei die Zeit, in welcher sich die Inseln von den exokrinen Zellen lösen entsprechend bestimmt. Das durch die Lösungsmischung aufgeweichte Pankreas wird hierbei, dass heisst, während der Digestionsphase in Fragmente zerkleinert (Ruptur) und anschliessend zur Gewinnung der Inseln mit entsprechenden Mitteln gefiltert (EP-A 0 191 613).

Bei diesem an sich traumatischen Verfahren wird relativ viel Gewebe und somit auch funktionsfähiges Zellenmaterial von den bekanntlich wenig zur Verfügung stehenden Organen, insbesondere humanen Spenderorganen zerstört. Zudem weist das in der nach diesem Verfahren gewonnenen Suspension verbleibende, weiterverwendbare Zellenmaterial nicht den Reinheitsgrad auf, wie er für eine erfolgreiche Transplantation oder dergleichen erforderlich ist.

Das der Erfindung zugrundeliegende Problem, nämlich aus der ohnehin schon geringen Anzahl zur Verfügung stehenden Organe den relativ geringen Anteil endokrinen Zellen-oder Inselmaterials unter Erreichung eines optimalen Reinheitsgrades zu erhöhen, wird gemäss dem erfindungsgemässen Verfahren dadurch gelöst, dass

a) das Spenderorgan während einer Perfusionsphase unter gleichen sterilen und temperaturabhängigen Verhältnissen wie beim Spender mit einer gemischten Blut-Substanz künstlich durchblutet wird,

b) das Spenderorgan während einer Dissolutions-oder Digestionsphase in ein mit Collagenase-Flüssigkeit gefülltes Gefäss solange eingetaucht bleibt, bis eine Abscheidung des zu entfernenden Fasermaterials (Pankreasgang, Gefässe etc.) erfolgt und der verbleibende Rest eine homogene Fragment-Suspension bildet,

c) die Fragment-Suspension während einer Inkubationsphase zur Gewinnung eines "Absatzes" (Sediment) aus exokrinem Zellmaterial und endokrinen Zellen zentrifugiert wird, und

d) während einer Insel-Isolationsphase die toten, exokrinen Zellenanteile durch Zentrifugieren und Ausspülen von den lebenden, endokrinen Zellen (Inseln) getrennt und entfernt werden.

Die erfindungsgemässe Einrichtung zur Durchführung des Verfahrens ist dadurch gekennzeichnet, dass die Einrichtung als Perfusionseinrichtung ausgebildet ist und einen Organ-Aufnahmebehälter aufweist, welcher für eine dem Organspender entsprechende künstliche Durchblutung des entnommenen Organs mit einer Zuführleitung mit dem Oxygenatorgerät und einer Rücklaufleitung zur Aufbereitung der verbrauchten Blut-Substanz mit dem Wärme-und Gastauscher verbunden ist.

Weitere Merkmale der Erfindung ergeben sich aus der folgenden Beschreibung in Verbindung mit der Zeichnung und den weiteren Patentansprüchen.

Die Erfindung wird nachstehend in Verbindung mit der Zeichnung beschrieben. Es zeigt:

Fig. 1 eine schematische Darstellung einer Organ-Perfusionseinrichtung, und

Fig. 2 eine schematisch dargestellte Vorrichtung zur Durchführung einer Organ-Dissolutionsphase.

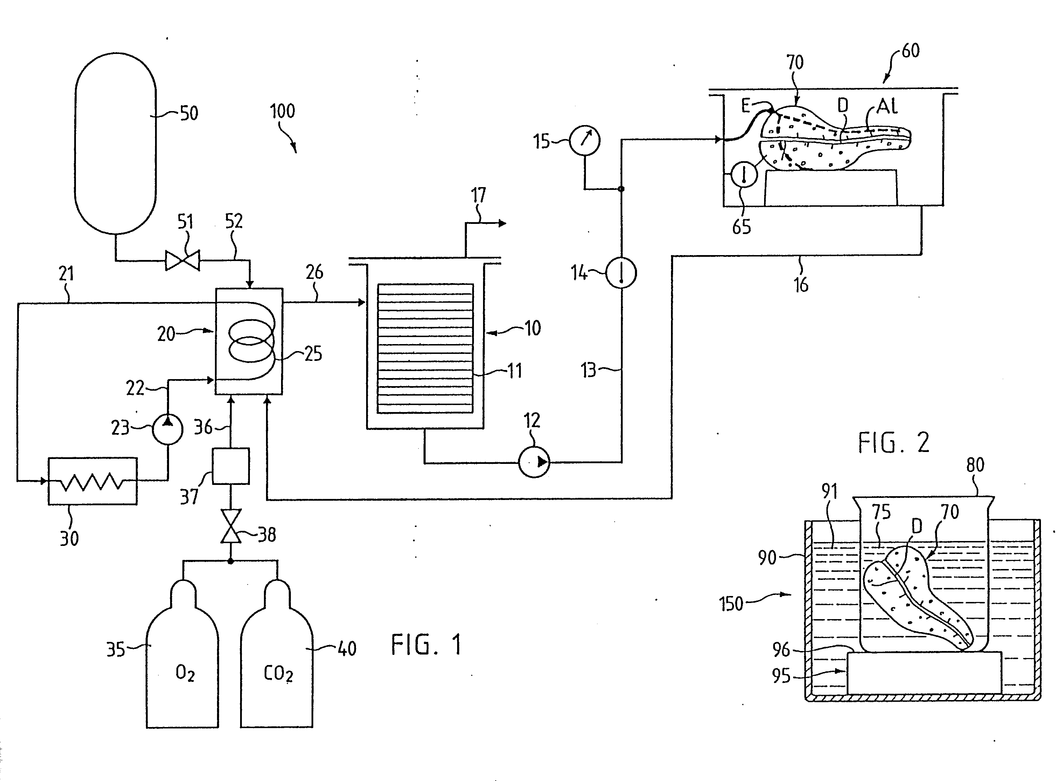

Fig. 1 zeigt eine schematisch und als Blockschaltbild dargestellte Perfusionseinrichtung, welche in ihrer Gesamtheit mit 100 bezeichnet und zur künstlichen Durchblutung eines Spenderorgans, insbesondere einer Bauchspeicheldrüse ausgebildet ist.

Die weitgehend nach dem Prinzip einer Herz-Lungen-Maschine arbeitende Perfusionseinrichtung 100 umfasst im wesentlichen ein Oxygenatorgerät 10 mit zugeordnetem Wärme-und Gastauscher 20, eine Heizvorrichtung 30 mit nicht dargestelltem Temperatur-Regelorgan, zwei Flüssiggasbehälter 35 und 40, einen Vorratsbehälter 50 sowie einen Aufnahmbehälter 60 für das mit 70 bezeichnete Spenderorgan.

Der Vorratsbehälter 50 ist über eine Leitung 52 mit zugeordnetem Absperrventil 51 mit dem Wärme-und Gastauscher 20 verbunden. In dem Wärme-und Gastauscher 20 ist eine entsprechend spiralförmig ausgebildete Heizschlange 25 angeordnet, welche über einer Zuführ-und Rücklaufleitung 21,22 mit zwischengeschalteter Druckpumpe 23 mit der Heizvorrichtung 30 wirkverbunden ist, wobei die Teile 21,23,22,25 und 30 ein geschlossenes Kreislauf-System bilden.

Der Wärme-und Gastauscher 20 steht weiterhin über eine Leitung 26 mit dem Oxygenatorgerät 10 sowie über eine Leitung 36 unter Zwischenschaltung eines Druckreglers 37 und eines Absperrventils 38 mit den beiden Behältern 35 und 40 in Verbindung.

Das mit einem einsetzbaren Filter 11 versehene Oxygenatorgerät 10 steht unter Zwischenschaltung einer Förderpumpe 12 und einem Temperaturfühler 14 über eine Zuführleitung 13 mit dem Aufnahmebehälter 60, und dieser über eine Rücklaufleitung 16 mit dem Wärme-und Gastauscher 20 in Verbindung. An der Zuführleitung 13 ist weiterhin ein Druckmessgerät 15 angeordnet und an dem Oxygenatorgerät 10 eine Entlüftungsleitung 17 vorgesehen.

Der Aufnahmebehälter 60 ist zur strengen, sterilen Aufnahme des Spenderorgans 70 ausgebildet, wobei das Spenderorgan 70 - im schematisch dargestellten Beispiel eine von einem Kanal D (Ductus pancreatius) durchdrungene Bauchspeicheldrüse (Pankreas) - vorzugsweise mit der schematisch dargestellten "Arterial lienalis" AL (gestrichelte Linie), durch welche das Spenderorgan 70 hauptsächlich mit Blut versorgt wird, am Eingang E der Zuführleitung 13 mit nicht näher dargestellten Mitteln angeschlossen wird.

Das bei der künstlichen Durchblutung im wesentlichen von den Venen (nicht dargestellt) des Pankreas abgegebene, verbrauchte Blut (Blut-Substanz) wird von der entsprechend am Aufnahmebehälter 60 angeordneten und angeschlossenen Rücklaufleitung 16 dem Wärme-und Gastauscher 20 zugeführt. Zur Ueberwachung und Regelung der Blut-und Organtemperatur im Aufnahmebehälter 60 ist ein entsprechend angeordnetes und regulierend wirkendes Fühlerelement 65 vorgesehen.

In Fig.2 ist in schematischer Schnittansicht eine Vorrichtung 150 zur Durchführung einer Dissolutionsphase des Spenderorgans 70 dargestellt, welche Vorrichtung 150 im wesentlichen einen Behälter 90 mit darin angeordnetem, eine Auflage- oder Stellfläche 96 aufweisenden Auflageelement 95 umfasst.

Der mit Wasser 91 gefüllte Behälter 90 dient bei der Dissolutionsphase als Wasserbad für das in einem becherförmigen, sterilen Gefäss 80 befindliche Spenderorgan 70, wobei das Gefäss 80 zur Dissolution des Spenderorgans 70 mit einer entsprechenden Flüssigkeit 75, vorzugsweise mit Collagenase-Flüssigkeit gefüllt ist.

Der Behälter 90 ist zur Temperatur-Konstanthaltung des Wasserbades 91 mit nicht näher dargestellten Mitteln (Aufheizelemente) versehen.

Das erfindungsgemässe Verfahren zur Gewinnung des endokrinen Zellenanteils (Inseln) aus einem Spenderorgan, beispielsweise aus einer Bauchspeicheldrüse wird nachstehend beschrieben:

Die von einem Spender unter absolut sterilen Voraussetzungen entnommene Bauchspeicheldrüse 70 (nachstehend Pankreas genannt) wird unter gleichen organischen Voraussetzungen wie beim Spender zur Durchblutung in den Behälter 60 eingebracht und wie bereits vorstehend beschrieben entsprechend an die Zuführleitung 13 angeschlossen und anschliessend der Behälter 60 mit nicht näher dargestellten Mitteln steril verschlossen. so dass die Perfusionseinrichtung 100 in Betrieb genommen werden kann.

Bei diesem Vorgang, d. h., beim Anfahren der Perfusionseinrichtung 100 wird zur Vorbehandlung des Pankreas 70 aus dem Vorratsbehälter 50 eine entsprechende Blut-Substanz - bestehend aus Anteilen von Blut, Selenomethionin, Natriumbicarbonat, Heparin und Ringerlactat - dem Wärme-und Gastauscher 20 zugeführt. Zur Aufsättigung und zum Gasaustausch wird gleichzeitig der Blut-Substanz über die am Wärme-und Gastauscher 20 angeschlossene Leitung 36 dauernd ein Gemisch - Luft-Sauerstoff und Kohlendioxyd - zugeführt.

Von dem Wärme-und Gastauscher 20 gelangt die Blut-Substanz über die Leitung 26 in das Oxygenatorgerät 10, wird dort in an sich bekannter Weise entsprechend aufbereitet (z.B. Entschäumung durch den Filter 11), anschliessend mittels der Pumpe 12 über die Leitung 13 durch den Pankreas 70 und danach über die Leitung 16 dem Wärme-und Gastauscher 20 zur Wiederaufbereitung zugeführt.

Der vorstehend beschriebene, erste Verfahrensschritt (Perfusionsphase) entspricht im wesentlichen einer künstlichen Durchblutung des Pankreas 70 mit der bestimmten Blut-Substanz und erfolgt während einer Zeitdauer in der Grössenordnung von 1,0 bis 5,0 Stunden, vorzugsweise aber während 1,5 bis 3,0 Stunden bei 37° Celsius unter strengen, sterilen Bedingungen.

Versuche haben gezeigt, dass insbesondere die in der Blut-Substanz enthaltenen Anteile "Selenomethionin" in relativ kurzer Zeit eine selektive Veränderung an den exokrinen Zellen (etwa 97-98% des Pankreas) bewirken, während die endokrinen Zellen oder Inseln (etwa 2-3% des Pankreas) keine Veränderungen zeigten.

Für den nächsten, zweiten Verfahrensschritt (Dissolutions-oder Digestionsphase) wird dem aus dem Behälter 60 entnommenen Pankreas 70 eine Collagenase-Flüssigkeit, vorzugsweise intraductal, d.h., in den mit D bezeichneten Pankreasgang injiziert. Die zu injizierende Collagenase ist in Abhängigkeit vom Pankreasgewicht und liegt in der Grössenordnung zwischen 0,25 bis 2,0 Gramm.

Der auf diese Weise vorbereitete Pankreas 70 wird anschliessend, wie in Fig. 2 dargestellt, in das mit Collagenase-Flüssigkeit 75 gefüllte Gefäss 80 eingebracht und danach das Gefäss 80 mit dem Pankreas 70 auf das im Behälter 90 vorgesehene Auflageelement 95 abgestellt. Die in dem Gefäss 80 eingefüllte Collagenase 75 ist bei diesem Vorgang bereits auf 37° Celsius vorgewärmt, während das im Behälter 90 enthaltene Wasserbad 91 mit nicht dargestellten Mitteln auf eine Temperatur von 37-39° Celsius und damit auch die Collagenasetemperatur konstant gehalten wird.

Versuche haben gezeigt, dass sich hierbei nach einer Zeitdauer von etwa 10 bis 25 Minuten feinste Gewebe-Fragmente von dem Pankreas-Gang (Duct) und den Pankreas-Gefässen lösen, so dass dieses im wesentlichen zusammmenhängende Fasermaterial (Pankreas-Gang, Gefässe und kollagene Fasern) mit geeigneten Mitteln (Pinzette) gesamthaft aus dem Gefäss 80 entfernt werden konnte. Nach weiteren 15 bis 30 Minuten bildete sich eine homogene Suspension von Fragmenten gleicher Grösse, welche sich am Boden des Gefässes 80 absetzte.

In einem weiteren, dritten Verfahrensschritt (Inkubationsphase) wird die im zweiten Verfahrensschritt gewonnene Fragment-Suspension mehrmals zentrifugiert und dabei mit geeigneter Lösung solange gewaschen, bis die Collagenase entfernt und nicht mehr vorhanden ist.

Der so gewonnene "Absatz" (Sediment) von etwa 30 bis 40ml feinster Fragmente (Grössenordnung ca. 0,5-1,0mm) besteht aus exokrinen Zellen mit über 90% Anteilen sowie aus endokrinen Zellen oder Inseln mit etwa 2 bis 3% Anteilen.

Zur Eliminierung des exokrinen Zellenmaterials wird das gewonnene Sediment aus exokrinen und endokrinen Zellen mit folgender Konzentrations-Zusammensetzung: - Zellenmaterial, Kulturmedium, Serum, Selenomethionin, Enzyme und Luft - entsprechend kultiviert. Das Mengenverhältnis der einzelnen vorstehend erwähnten Elemente für ein 500ml Inkubations-Gefäss (nicht dargestellt) wird vorzugsweise wie nachstehend aufgeführt gewählt:

a) 2,5-10,0 ml Zellmaterial (Sediment),
b) 175-250 ml Kulturmedium (RPMI 1640),
c) 10-40 ml fötales Kälberserum (FCS),
d) 40-100 mg Selenomethionin (Feststoff), und
e) 10-40 mg Desoxyribonuclease (Feststoff).

Die Füllmenge der vorstehenden Elemente a) bis e) beträgt max. 50% des Gefäss-Inhaltes und der Restinhalt ist Luft (Bedingung).

Zur Kultivierung des Zellenmaterials wird das mit den Elementen a) bis e) gefüllte Inkubations-Gefäss auf einen an sich bekannten Rollenmischer (nicht dargestellt) gelegt und der Rollenmischer betätigt, so dass der Gefäss-Inhalt in gemischtem Zustand gehalten wird. Im Verlauf der Kultivierung lösen sich dabei die exokrinen Zellen auf oder sterben ab, während die endokrinen Zellen (Inseln) bestehen bleiben und (natürlich) weiterleben. Die vorstehend beschriebene Zellmaterial-Kultivierungsdauer hängt im wesentlichen von der Selenomethionin-Konzentration ab und liegt in der Grössenordnung zwischen 18 bis 30 Stunden.

In einem vierten und letzten Verfahrensschritt (Insel-Isolation) zur Gewinnung der endokrinen Zellen oder Inseln, wird die im Verlauf der Kultivierung gewonnene und in Reagenzgefässe eingebrachte Kultur durch Zentrifugieren in einer nicht dargestellten Zentrifuge von den Kulturmedien entsprechend freigewaschen. Dabei unterteilt sich die Kultur im wesentlichen in zwei Schichten, und zwar

1. in endokrine Zellen oder Inseln die als Sediment am Boden haften und leben, sowie

2. in aufgelöste exokrine, toten Zellteile (weiss) von exokrinen Zellen, wobei die toten, weissen Zellteile durch Aüsspülen mittels einer geeigneten Lösung entfernt werden und die endokrinen Zellen (Inseln) als sogenannter "Absatz" (Sediment) am Boden des einzelnen nicht dargestellten Reagenzgefässes haften.

Die anhand der vorstehend in Verbindung mit der Perfusionseinrichtung 100 und den nicht dargestellten Mitteln beschriebenen Verfahrensschritte - Perfusionsphase, Dissolutions-oder Digestionsphase, Inkubationsphase und Isolationsphase - atraumatisch gewonnenen endokrinen Zellen oder Inseln können mit einer geeigneten Lösung in Supension gebracht werden.

Das gewonnene Inselpräparat wird vorzugsweise zur Wiederaufnahme der normalen Stoffwechsel-Funktion eines entsprechenden Organs verwendet, und kann beispielsweise transplantiert oder injiziert werden.

## Ansprüche

1. Verfahren zur Gewinnung endokriner Zellen oder Inseln aus steril entnommenen Spenderorganen, insbesondere aus einer Bauchspeicheldrüse (Pankreas), dadurch gekennzeichnet, dass

a) das Spenderorgan (70) während einer Perfusionsphase unter gleichen sterilen und temperaturabhängigen Verhältnissen wie beim Spender mit einer gemischten Blut-Substanz künstlich durchblutet wird,

b) das Spenderorgan (70) während einer Dissolutions-oder Digestionsphase in ein mit Collagenase-Flüssigkeit (75) gefülltes Gefäss (80) eingetaucht bleibt, bis eine Abscheidung des zu entfernenden Fasermaterials (Pankreasgang, Gefässe etc.) erfolgt und der verbleibende Rest eine homogene Fragment-Suspension bildet,

c) die Fragment-Suspension während einer Inkubationsphase zur Gewinnung eines Absatzes (Sediment) aus exokrinem Zellmaterial und endokrinen Zellen zentrifugiert wird, und

d) während einer Insel-Isolationsphase die toten, exokrinen Zellenanteile durch Zentrifugieren und Ausspülen von den lebenden, endokrinen Zellen (Inseln) getrennt und entfernt werden.

2. Verfahren nach Anspruch 1, dadurch gekennzeichnet, dass das Spenderorgan (70) mit einer Blut-Substanz, bestehend aus Anteilen von Blut, Selenomethionin, Natriumcarbonat, Heparin und Ringerlactat während einer Zeitdauer von 1,0 bis 5,0 Stunden, vorzugsweise während 1,5 bis 3,0 Stunden bei gleichbleibender Temperatur von 37° Celsius künstlich durchblutet wird.

3. Verfahren nach Anspruch 2, dadurch gekennzeichnet, dass die in der Blut-Substanz enthaltenen "Selenomethionin" Anteile so gewählt und bemessen sind, dass nach relativ kurzer Zeit, etwa nach 1,5 bis 3,0 Stunden, eine selektive Veränderung des Spenderorgans (70) erreicht wird.

4. Verfahren nach Anspruch 1 und 2, dadurch gekennzeichnet, dass das Spenderorgan (70) vor der Dissolutions-oder Digestionsphase zusätzlich mit einer Collagenase-Flüssigkeit, vorzugsweise intraductal (in den Pankreasgang D) injiziert wird.

5. Verfahren nach Anspruch 1 und 4, dadurch gekennzeichnet, dass die Dissolutions-oder Digestionsphase während einer Zeitdauer von etwa 25 bis 60 Minuten durchgeführt wird.

6. Verfahren nach Anspruch 1, dadurch gekennzeichnet, dass das gewonnene Sediment mit folgender Konzentration-Zusammensetzung
a) 2,5 bis 10 ml Zellmaterial (Sediment),
b) 175 bis 250 ml Kulturmedium (RPMI 1640),
c) 10 bis 40 ml fötales Kälberserum (FCS),
d) 40 bis 100 mg Selenomethionin (Feststoff), und
e) 10 bis 40 mg Desoxyribonuclease (Feststoff) während einer Zeitdauer von etwa 8 bis 30 Stunden mit entsprechenden Mitteln kultiviert wird.

7. Verfahren nach Anspruch 6, dadurch gekennzeichnet, dass die Füllmengen der Elemente a) bis e) im Maximum 50% eines Gefäss-Inhalts betragen.

8. Verfahren nach einem der Ansprüche 1 bis 7, dadurch gekennzeichnet, dass bei den jeweiligen Phasen Selenomethionin und/oder vergleichbare chemische Verbindungen basierend auf Methionin sowie dessen Derivate verwendet werden.

9. Einrichtung zur Durchführung des Verfahrens nach einem der vorgehenden Ansprüche 1 bis 7, welche Einrichtung ein Oxygenatorgerät (10), einen Wärme-und Gastauscher (20), eine Heizvorrichtung (30) sowie zwei Flüssiggas-Behälter (35,40) für eine ständige Gas-Gemischzufuhr zum Wärme-und Gastauscher umfasst, dadurch gekennzeichnet, dass die Einrichtung aus Organ-Perfusionseinrichtung 100 ausgebildet ist und einen Organ-Aufnahmebehälter (60) aufweist, welcher für eine dem Organspender entsprechende künstliche Durchblutung des Organs (70) mit einer Zuführlei tung (13) mit dem Oxygenatorgerät (10) und einer Rücklaufleitung (16) zur Aufbereitung der Blut-Substanz mit dem Wärme-und Gastauscher (20) verbunden ist.

10. Einrichtung nach Anspruch 9, dadurch gekennzeichnet, dass der Aufnahmebehälter (60) Mittel zum Anschluss der Zuführleitung (13) an die Arteria lienalis (AL) des Organs (70) aufweist, und dass die Rückführleitung (16) im unteren Bereich des Aufnahmebehälters (60) angeordnet ist.

11. Einrichtung nach Anspruch 9 und 10, dadurch gekennzeichnet, dass der Aufnahmebehälter (60) steril verschliessbar ausgebildet ist.

12. Verwendung der nach einem der vorstehenden Verfahrensansprüche 1 bis 8 gewonnenen endokrinen Inseln, dadurch gekennzeichnet, dass die Inseln mit einer geeigneten Lösung (Kochsalzlösung) in Suspension gebracht und zur Wiederaufnahme der normalen Stoffwechsel-Funktion eines Organs verwendet werden.

13. Verwendung nach Anspruch 12, gekennzeichnet durch eine Injektion oder Transplantation der gewonnenen endokrinen Inseln.

14. Verwendung nach Anspruch 12 und 13, gekennzeichnet durch eine intraportale oder transcutane Transplantation der gewonnenen endokrinen Inseln in die Leber.

FIG. 1

FIG. 2

0 287 896

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 191 613 (McDONNELL DOUGLAS CORP.) <br> * Ansprüche 1,2,9,10; Seite 1, Zeilen 6-18; Seite 2, Zeile 24 - Seite 3, Zeile 3 * <br> --- | 1-14 | A 61 K 35/39 <br> A 01 N 1/00 <br> A 61 M 1/00 |
| Y | BE-A- 643 051 (UNION CARBIDE CORP.) <br> * Seite 3, Zeilen 5-26; Seite 4, Zeile 11 - Seite 5, Zeile 26; Seite 9, Zeilen 1-16; Seite 9, Zeile 24 - Seite 10, Zeile 7; Seite 13, Zeilen 12-26; Seite 14, Zeilen 14-17; Seite 15, Zeilen 14-21; Seite 17, Zeile 27 - Seite 19, Zeile 25 * <br> --- | 1,4-7, 12-14 | |
| Y | WO-A-8 301 196 (E. JOHANSSON) <br> * Ansprüche 1,7 * <br> --- | 2,3,8 | |
| Y | DE-A-2 046 813 (BAXTER LABORATORIES) <br> * Ansprüche 1-10,18; Seite 2, Zeilen 10-20; Seite 5, Zeilen 3-11; Seite 6, Zeilen 16-26 * <br> --- | 9-11 | **RECHERCHIERTE SACHGEBIETE (Int. Cl.4)** |
| A | GB-A- 919 829 (P. NIEHANS) <br> * Ansprüche 1-14; Seite 2, Zeile 113 - Seite 3, Zeile 39; Beispiel V * <br> ----- | 1,12-14 | A 61 K <br> A 01 N |

Der vorliegende Recherchenbericht wurde für alle Patentansprüche erstellt

| Recherchenort | Abschlußdatum der Recherche | Prüfer |
|---|---|---|
| DEN HAAG | 22-07-1988 | RYCKEBOSCH A.O.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

------------------------------------------------------------
& : Mitglied der gleichen Patentfamilie, übereinstimmendes Dokument

EPO FORM 1503 03.82 (P0403)